# EUROPEAN PATENT APPLICATION

(11) **EP 4 162 949 A2**
(43) Date of publication of application: **12.04.2023**
(21) Application number: 21761969.1
(22) Date of filing: 31.05.2021
(51) Int. Cl.: A61K 39/00

(54) **INORGANIC NANOPARTICLE-BASED VACCINE COMPOSITIONS FOR CANCER TREATMENT**

(30) Priority: 09.06.2020 CU 20200032
(71) Applicant: Centro de Inmunologia Molecular, Playa, Habana 11300 (CU); Universidad de La Habana, La Habana 10400 (CU)
(72) Inventor: GONZÁLEZ RUIZ, Gustavo, La Habana 10900 (CU); GONZÁLEZ MARTÍNEZ, David Alejandro, La Habana 10900 (CU); BORDALLO LEÓN, Fernando, La Habana 17100 (CU); SÁNCHEZ RAMÍREZ, Belinda, La Habana 11300 (CU); LEÓN MONZÓN, Kalet, La Habana 17100 (CU); ECHEVERRÍA LUNA, Yerandy, La Habana 10400 (CU); LUZARDO LORENZO, Maria del Carmen, La Habana 10400 (CU); CRUZ RODRÍGUEZ, Mabel, La Habana 11300 (CU); GONZÁLEZ PALOMO, Adys, La Habana 10900 (CU); SANTO TOMÁS POMPA, Julio Felipe, La Habana 10900 (CU); GARCÍA ARTALEJO, Judey Aymed, La Habana 11600 (CU); RUIZ CASTRO, Elaine, La Habana 17100 (CU); LÓPEZ MATILLA, Lien, La Habana 10700 (CU)
(74) Representative: V.O.
(86) International application number: PCT/CU2021/050005
(87) International publication number: WO 2021/259397

(57) **Abstract**

The present invention is related to biotechnology, particularly to the field of human health. It provides new vaccine compositions that comprise as active principle a system that contains the recombinant human EGF, or peptides thereof, and a carrier protein or peptide, bound to a nucleus constituted by inorganic nanoparticles, with nanometric or submicrometric scale dimensions. These vaccine compositions are useful for the chronic treatment of cancer and have as advantages that their administration does not result in the appearance of adverse effects at the injection site and that they do not accumulate in the body.

## Description

### SCOPE OF THE TECHNIQUE

The present invention is related to biotechnology, specifically to the field of human health. Particularly it describes new vaccine formulations that contain nanoparticulated systems that induce an immune response against the epidermal growth factor, and are therefore useful as cancer therapies.

### BACKGROUND

Tumors in epithelial origin tissues generally overexpress the receptor of the epidermal growth factor (EGF) on their surface. The binding of the autologous EGF to its receptor (EGFR) activates mechanisms in the tumor that lead to its growth and to its escape from the protection that the immune system confers. Based on this knowledge, from the 1990s on, the EGF was identified as a therapeutic target for the treatment of lung cancer and other epithelial origin cancers, in which the binding of the EGF to its receptor is relevant.

Many researches aimed at assessing passive immunotherapy with EGFR-targeting antibodies have been conducted. Previously it was demonstrated that the specific recognition of the receptor by antibodies of murine origin inhibits the mitogenic stimulation of the malignant cells (Sato J.D. et al., Methods in Enzymology (1987) 146:63-81). In another study, published in US 5,891,996, patent, chimerical and humanized monoclonal antibodies that can play a diagnostic or therapeutic role in tumors with overexpression of the EGFR are described.

The use of active immunotherapies has been expanding rapidly in the last few years, due to their potential for fighting against cancer and turning it into a chronic manageable disease. Unlike passive therapies, active therapies are based on stimulating the immune system to act against the tumor or other elements that propitiate their development. Among other advantages, they reduce toxicity since the doses required contain only small quantities of the active principles.

The formulation referred to in US 5,894,018 patent claims a vaccine composition that produces an immune response against the autologous EGF. It comprises the autologous EGF conjugated to a carrier protein (cholera toxin B subunit, tetanus toxoid, a monoclonal antibody or a protein from the outer membrane of *Neisseria meningitidis*), adjuvanted with aluminum hydroxide. On the other hand, US 8,778,879 patent describes a vaccine composition for therapeutic use in cancer patients that has as active principle the chemical conjugate of recombinant human EGF (rhEGF) with recombinant protein P64k. It describes a procedure for the purification of the chemical conjugate that increases its purity and immunogenic activity. This composition contains an adjuvant that can be hydroxide aluminum or Montanide.

Later clinical studies demonstrated that the therapeutic vaccine derived from the previously mentioned inventions, was safe and effective for the treatment of advanced-stage Non-Small Cell Lung Cancer (NSCLC) (Rodriguez P. C. at al., Clinical Cancer Research (2016), 22 (15): 3782-3790). However, it was evidenced the patients that achieved high survival rates (five years or more) suffer from injury in the muscles (deltoid and gluteal) of the injection sites, resulting from the accumulation of the mineral oil the adjuvant contains. For that, eventually leads to some patients having to interrupt the treatment or to the need of inoculating the product into muscles more distant from the lymphoid organs.

Immunologic adjuvants arose from the need of increasing the immunogenicity of the vaccines developed from recombinant proteins. The first adjuvant used was the double sulphate of aluminum, in 1926. Subsequently diverse inorganic adjuvants from aluminum, calcium, magnesium, iron, zinc, zirconium, cerium, beryllium and silicon were assessed. However, only the use of aluminum and calcium salts has been approved in humans (Paneque-Quevedo A., Applied Biotechnology (2013) 30: 243-249). In all the previous studies conducted, inorganic adjuvants were used in combination with heterologous antigens.

The aluminum hydroxide and the aluminum phosphate adjuvants are not very suitable for chronic treatments, since aluminum is toxic and poorly metabolized by the human body. The impact of the aluminum accumulation on human health has been well documented, specifically on the following systems: central nervous, musculoskeletal, respiratory, cardiovascular, endocrine, urinary and reproductive (Nayak P., Environmental Research Section TO (2002), 89: 101-115; Verstraeten S. A. et al., Arch Toxicol (2008) 82: 789-802; Flaten T. P., Brain Research Bulletin (2001) 2: 187-196).

On the other hand, calcium phosphates (hereinafter CaP) are inorganic substances which have been demonstrated to be useful as immunologic adjuvants. In US patent 2,967,802, the mixture of an erysipelas antigen with a hydrated CaP gel is claimed. In studies and subsequent patents prophylactic vaccines against infections caused by several pathogenic and allergenic microorganisms adsorbed in CaP were developed (US 3,608,071, US 4016252; US 4350686; US 20120121714; Coursaget, P. et al., Infect Immun (1986) 51(3): 784-787).

As is the case with hydroxide and aluminum phosphate, the CaP adjuvants immunologic effect is based on the formation of a depot of the antigen that is slowly released, which allows for a prolonged time of presentation to the immune system sufficient to generate a response against the inoculated antigen. US 8,333,996 patent claims the development of immunogenic systems formed by the adsorption of antigens such as: *Bordetella pertusis,* allergens, human immunodeficiency (HIV-2) inactivated viruses, Keyhole limpet hemocyanin (KLH), vaccines against diphtheria, tetanus toxoid, streptococci, attenuated bacteria or viruses, polio and hepatitis A and B. They also include DNA and cytokine adsorption, such as the granulocyte-macrophage colony-stimulating factor (GM-CSF).

In US 6,355,271 patent a method for preparing particles of CaP with diameters between 300 and 4000 nm is claimed, which is based on the aqueous mixture of calcium chloride, sodium phosphate and sodium citrate for the adsorption of HSV-1, HSV-2 and EBV viruses, DNA, as well as ovalbumin and *Mycobacterium tuberculosis* antigens.

Recent studies has assessed the use of CaP as vaccine adjuvant or for the release of diverse medicines. Among them, those assessing the development of particulate hydroxyapatite systems (Ca₁₀(PO₄)6(OH)₂, hereinafter referred to as HAp), preferably in the form of nanoparticles, are relevant. This substance is the main inorganic component of the bone tissue, therefore, its biocompatibility is guaranteed. In addition, the amorphous or low crystallinity HAp are biodegraded in contact with the biological medium. The reduction of the size of the particles and the selected material give it superior properties as an adjuvant, as compared to aluminum hydroxide. They produce a more balanced Th1/Th2 response and less generation of IgE-type antibodies (Qing, H. et al. Clinical and Diagnostic Laboratory Immunology (2000) 7(6): 899-903; Jones, S. et al., Vaccine (2014) 32: 4234-4242; Lin, Y. et al. Expert Review of Vaccines (2017), 16(9): 895-906. Also, this substance reduces the adverse effects at the injection site since it is biocompatible and in its amorphous form biodegradable.

WO 2005084637 patent claims a system comprising: a nucleus of CaP nanoparticle, a biologically active macromolecule encapsulated in the particle of the nucleus and a surface modifying agent. The biologically active macromolecule can be a protein, a polypeptide, a polysaccharide, a nucleic acid, a polynucleotide, a lipid or a carbohydrate.

On the other hand, WO 2003051394 invention shows a HAp nanoparticulated with different types of coating in one single formulation for use as adjuvant. In it, nanoparticles are employed as carriers of native or recombinant antigens or other pharmacological agents to be applied to the surfaces of the mucosas.

Other studies, claim developments and applications of the CaP in general and specifically of HAp nanoparticles as immunologic adjuvants or active substances carriers, to be employed for the treatment of infectious or immune system diseases, including cancer (US 5,443,832; US 6,355,271; US 6,767,550; US 20020068090; US 7,776,600; WO 2017025359).

None of the previous reports describes vaccine compositions that induce a specific immune response against the EGF or other autologous proteins, constructed on the surface of CaP particles, from their chemical binding with rhEGF molecules and a carrier protein independently, or by the conjugation of both.

The novelty of this invention consists in providing new vaccine compositions for the chronic treatment of cancer, formed by a nucleus of biodegradable inorganic nanoparticles, chemically linked to autologous antigens. By means of the bound of CaP particles, specifically HAp, of nanometric or submicrometric scale dimensions, with the rhEGF or peptides thereof and another protein or carrier peptide able to generate an immune response against the EGF. These new compositions are superior to the existing ones that raise a response against the EGF, in that they do not produce adverse effects at the injection site and they are not accumulated in the body. Also, they are dispensed in one single vial so they do not require additional procedures such as preparing mixtures or emulsions at the time of use.

### BRIEF DESCRIPTION OF THE INVENTION

In one embodiment, the present invention relates to vaccine compositions to induce an immune response against the EGF. Characterized in that it comprises as active principle a system containing the rhEGF or peptides thereof and a carrier protein or peptide linked to a nucleus formed by biodegradable inorganic nanoparticles that can be salts, calcium oxides or hydroxides, iron, zinc, magnesium, zirconium, cerium, beryllium, silicon, or the mixture of two or more of them. Preferably, the inorganic nucleus is composed of CaP, more particularly of HAp type. This HAp is preferably amorphous or of low cristallinity and is partially or totally coated with an organic ligand, particularly sodium citrate.

Additionally, the protein or peptide carrier is selected from the group comprising: cholera toxin B subunit, tetanus toxoid, KLH and the P64k of *Neisseria meningitidis.*

Also, the active principle of the vaccine compositions of the present invention is on the surface of the HAp nanoparticles, bound by means of one of the following methods:
- Covalent bond of the chemical conjugate of the rhEGF or peptides thereof and the carrier protein or peptide with the HAp nanoparticle (HAp-rhEGF-rP64k).
- Covalent bond of the rhEGF or peptides thereof and the carrier protein or peptide with the HAp nanoparticle in an independent way (HAp-PI).
- Successive covalent bond of the rhEGF or peptides thereof and the carrier protein or peptide with the HAp nanoparticle.
- Multiple conjugation between the rhEGF or peptides thereof and the carrier protein or peptide on the surface of the HAp (HAp-CM).
- Encapsulation or physical adsorption of the rhEGF and the carrier protein or peptide on the surface of the HAp.

Another aspect of the present invention contemplates the combination of the vaccine compositions herein described with adjuvants selected from the groups comprising: incomplete Freund's adjuvants, squalene-based adjuvants, synthetic origin adjuvants, mineral origin adjuvants, vegetable origin adjuvants, animal origin adjuvants, particulate proteic adjuvants and liposomes.

In an additional embodiment, the object of the present invention is the use of the vaccine compositions herein described for the chronic treatment of cancer.

It is a further object of the present invention to provide a treatment method for a subject in need thereof that comprises the administration of a therapeutically effective amount of the vaccine compositions herein described. This method is particularly characterized in that it maintains the induction of the immune response achieved with another vaccine composition against the EGF.

### DETAILED DESCRIPTION OF THE INVENTION

The present invention comprises the synthesis of biodegradable inorganic nanoparticles, coated with organic ligands, their chemical activation, as well as their binding by means of covalent bonds or electrostatic interactions with an antigen that induces a specific immune response against the EGF and their construction on the surface of this particle.

### Obtaining and activation of biodegradable inorganic nanoparticles

The present invention comprises the formation of biodegradable inorganic particles of nanometric, submicrometric or micrometric scale sizes. These particles are coated with an organic ligand, preferably sodium citrate and are obtained by precipitation, although this method should not be construed as limiting the scope of the present invention.

The biodegradable particles have an inorganic nucleus formed by salts, oxides or calcium hydroxides, iron, zinc, magnesium, zirconium, cerium, beryllium, silicon, or the mixture of two or more of them. Preferably, they are formed by CaP, more preferably by amorphous HAp, or amorphous HAp with isomorphic substitutions of one or more metals.

For the formation of the inorganic particles, bearing sources of calcium and phosphate ions are used, also an organic ligand that confers a surface charge and functional group to them that allows for the chemical conjugation with biomolecules.

The particles used in the immunotherapeutic systems described in the present invention are characterized in that they have at least one of the following properties:
- They are formed by biodegradable inorganic particles that can have isomorphic substitutions of metal or other ions.
- They are obtained preferably by wet synthesis, more preferably by precipitation, although this method should not be construed as limiting the scope of the present invention.
- They have micrometric, submicrometric, nanometric sizes or combinations thereof.
- They have spherical, cylindrical, or plate-like morphology or combinations thereof, although these morphologies should not be construed as limiting the scope of the present invention.
- They are preferably coated with an organic ligand that confers a surface charge and functional groups to them that allows for their conjugation with antigens.

In order to obtain the inorganic particles described in this invention it is preferably required:
- To maintain a molar ratio of cation to anion between 1:4.
- To add sodium citrate or another organic ligand that has a similar effect to the reactants at a quantity sufficient to provide a molar ratio between 3:5 to the calcium ions content.
- To adjust the pH to 7-13, by means of the addition of an alkali, preferably ammonium hydroxide or sodium hydroxide.
- To perform the reaction for 1-4 h, preferably at room temperature (20±5°C).
- To perform the vacuum drying process at room temperature.

The inorganic particles obtained under the previously described conditions, partially or completely coated with an organic ligand that contains carboxylate groups, preferably sodium citrate is activated by means of the addition of a cross-linking reagent of the carbodiimide family or another one with similar functions. In this process, a reagent/particle mass ratio from 1 to 6 is used, preferably from 2 to 5 mg/mg. The suspension is kept under stirring at room temperature (20±5°C) for 1-4 h, preferably for 0.5-3 h. Subsequently, the activated particles are purified, preferably by means of processes of centrifugation or filtration.

By means of the previously described procedure, particles with predominant sizes of ≤200 nm, with their surface activated, that have the capacity to binding to proteins that have exposed functional groups are obtained. The above-mentioned characteristics makes them superior to previous developments from inorganic particles for the encapsulation or physical adsorption of active principles.

### Obtainment of the nanoparticulate system for the induction an anti-EGF response

The previously activated particles are mixed with the chemical conjugate from the recombinant proteins rhEGF and rP64k, at a rhEGF-rP64k/HAp mass ratio of 1-10, preferably at 2-7 mg/mg. The previously described reaction results in an amide type covalent bond between the amine groups (NH₂) of the conjugate and the carboxylate groups (COO⁻) on the surface of the particle. Electrostatic interactions also occur between the calcium groups remaining on the surface of the HAp and the rhEGF-rP64k conjugate, that it is negatively charged and has carboxylate groups exposed. Then the colloidal suspension is dispersed, preferably with EDTA and finally its pH is adjusted to values between 6.7 and 7.3.

In another realization variant, the chemical conjugate of recombinant proteins rhEGF and rP64k is substituted by the conjugate of the rP64k or another carrier protein with one or several peptides of the rhEGF.

The binding of the conjugates described in the present invention to the surface of the inorganic particles, which occurs by means of the previously specified procedure. Results in a novel system, the first of this type, for the treatment of cancer, that has a composition substantially different from that of the vaccine formulations described in US 5,894,018 and US 8,778,879 patents. Inoculation with this system produces an immune response that includes the generation of anti-EGF antibodies able to capture the circulating EGF and prevent its binding to its receptor on the surface of the tumor.

The system obtained by means of the covalent binding of the antigen to the inorganic particles is stable and can be stored for prolonged times periods, as required for parenteral formulations. This feature, makes it superior to immunotherapeutic or drug delivery systems, previously developed starting from inorganic particles, by means of the encapsulation or adsorption of active principles methods.

### Conformation of conjugates on the inorganic particles by means of covalent bonds and electrostatic interactions, for the induction of an anti-EGF response

The present invention comprises other methods for the construction of systems to induce an anti-EGF immune response, by means of covalent bonds and electrostatic interactions among its components, which are detailed next:
- *Binding of the rhEGF or peptides thereof and a carrier protein or peptides thereof to the inorganic nanoparticles.*

The particles obtained with the previously described methodologies, previously activated with a carbodiimide, preferably with 1-Ethyl-3-(3-dimethylaminopropyl) carbodiimide (EDC), are mixed with a sterile solution of phosphate saline buffer (PBS), pH 7±0, containing the rhEGF and rP64k at a rhEGF/rP64k molar ratio of 3 to 9, preferably between 4 and 8. Also, the HAp/rP64k and HAp/rhEGF mass ratios used are between 1-6, preferably between 2-5 mg/mg. Subsequently, the suspension is kept stirring at room temperature for 1-4 h, more preferably from 2-3 h.

In further realizations variants, the recombinant protein rP64k is substituted for a carrier protein as tetanus toxoid, KLH, or another. The present invention also comprises the substitution of rP64k protein by one or several immunogenic peptides of natural or synthetic origin.

In further realization variants, the rhEGF is substituted by one or several peptides thereof.

Using the method described above, a system in which the rhEGF and rP64k proteins are chemically and by means of electrostatic interactions bound to the nanoparticles, without being bound to each other, is obtained. The inorganic particle constitutes the structural element for linking that ensures the immunogenicity of the system. Furthermore, it reduces up to 40% the amount of rhEGF necessary for the formation of the system and reduces the risks associated with the toxicity of the glutaraldehyde used in the inventions US 5,894,018 and US 8,778,879 inventions.
- *Conjugation of the rhEGF or peptides thereof and a carrier protein or peptides thereof, by means of two reaction steps.*

The nanoparticles obtained with the previously methodologies described, previously activated with a carbodiimide, preferably EDC, are mixed with a solution of PBS, pH 7±0.3, containing rP64k at a mass ratio of 1-6, preferably between 2-5. The suspension is kept stirring at room temperature for 1-4 h, preferably for 2-3 h. Subsequently, the excess protein rP64k is removed, it is activated preferably with EDC, then the excess EDC is removed and it is mixed with the rhEGF at a molar ratio of 1-10 of rhEGF per rP64k, preferably at 2-6.

In further realization variants, tetanus toxoid, KLH or another carrier protein substitutes the recombinant protein rP64k. The present invention also comprises the substitution of the rP64k protein by one or several immunogenic peptides of natural or synthetic origin.

In further realization variants, the rhEGF is substituted by one or several peptides thereof.

This procedure generates a system in which the carrier protein is chemically or by means of electrostatic interactions bound to the biodegradable inorganic nanoparticles and also to the rhEGF. This method produces a rhEGF-rP64k conjugate formed by two layers of high purity proteins, without free rhEGF or rP64k. This conjugate is superior to the rhEGF-rP64k conjugate of US 5,894,018 and US 8,778,879 inventions, in which more than 40% of the rhEGF obtained is in its free form, which has no biological relevance. Also, it reduces the risks associated to the toxicity of the glutaraldehyde used in the two previously mentioned inventions.
- *Multiple conjugation of the rhEGF or peptides thereof and of a carrier protein or peptides thereof on the surface of the inorganic particle.*

The particles obtained with the previously described methodologies, previously activated with a carbodiimide, preferably EDC, are mixed with a solution of PBS, pH 7±0.3, containing rhEGF and rP64k. Both proteins react with the activated particles and with the excess EDC that stays in dissolution after the activation, binding the proteins to each other and to the surface of the particle. In order to obtain this result, a molar ratio of rhEGF/rP64k of 3-9 must been employed, preferably of 4-8. Also, the mass ratio particle/rP64k and particle/rhEGF to be used between 1-6, preferably between 2-5. They are kept stirring at room temperature for 1-4 h, more preferably for 2-3 h.

In further realization variants tetanus toxoid, KLH or another carrier protein substitutes the rP64k recombinant protein. The present invention also comprises the substitution of the rP64k protein by one or several immunogenic peptides of natural or synthetic origin.

In further realization variants, the rhEGF is substituted by one or several peptides thereof.

A complex matrix of rhEGF-rP64k conjugates is generated and these proteins linked with the ligand that coats the inorganic nanoparticle. Like previous realizations, this system generates an anti-EGF antibody immune response. It also reduces by up to 40% the quantity of rhEGF necessary for the formation of the system and reduces the risks associated to the toxicity of the glutaraldehyde used in US 5,894,018 and US 8,778,879 inventions.

### Conformation of systems for inducing the an anti-EGF response by means of encapsulation or adsorption on the inorganic particle

Although, the chemically bound systems obtained with the previously described procedures are more stable and appropriate for the vaccine formulations, the encapsulation and adsorption of the antigen are also useful procedures. For that reason, the present invention provides methods for synthesizing immunogens that have an inorganic nucleus and the rhEGF-rP64k conjugate or rhEGF and rP64k or peptides thereof, inside the particle or adsorbed on its surface.
- *Encapsulation of the rhEGF conjugate and a carrier protein or peptides thereof to the inorganic particle:*
   Sources bearing of calcium and phosphate ions are mixed in an aqueous dissolution, making sure the appropriate molar ratio of cation to anion of 1.4-3.5 is maintained. The rhEGF-P64k conjugate is added, at a mass ratio of rhEGF-rP64k/inorganic nanoparticle of 1-10, more preferably 2-7. Subsequently, ammonium hydroxide or sodium hydroxide are added until a pH from 8-10 is obtained and the reaction is kept under stirring for 1-4 h at room temperature. Once the reaction is concluded, it is preferably washed with purified water and separated by filtration. This procedure generates nanoparticles and microparticles that contain rhEGF-rP64k conjugates inside and on the surface bound by interactions among the positively charged groups of the particle and the carboxylate groups of the conjugate and among the negatively charged groups of the particle and the amine groups of the conjugate.

In further realizations variant, tetanus toxoid, KLH or another carrier protein substitutes the recombinant rP64k protein. The present invention also comprises the substitution of the rP64k protein by one or several immunogenic peptides of natural or synthetic origin.

In further realization variants, the rhEGF is substituted by one or several peptides thereof.
- *Encapsulation of the rhEGF or peptides thereof and a carrier protein or peptides thereof to the inorganic particle:*
   This procedure is similar to the previously described one, differing only in that rhEGF and rP64k are added to the dissolution, instead of the rhEGF-rP64k conjugate, in a 1-10 mass ratio, even more preferably 2-7. During this process a molar ratio of 1-10 of rhEGF per rP64k is maintained, more preferably 2-5 of rhEGF per rP64k. Once the reaction is concluded, it is preferably washed with purified water and separated by filtration. This procedure generates nanoparticles and microparticles that contain rhEGF-rP64k conjugates, inside and on the surface, bound by electrostatic interactions between the positively charged groups of the particle and the carboxylate groups of the conjugate and between the negatively charged groups of the particle and the amine groups of the conjugate.

In another realization variant tetanus toxoid, KLH or another carrier protein substitutes the recombinant protein rP64k. The present invention also comprises the substitution of the rP64k protein by one or several immunogenic peptides of natural or synthetic origin.

In further realization variants, the rhEGF is substituted by one or several peptides thereof.
- *Adsorption of the rhEGF conjugate and a carrier protein or peptides thereof on the inorganic particle:*
   The previously described particles coated with sodium citrate are mixed with a PBS dissolution, pH 7±0.3, containing the rhEGF-rP64k conjugate in a 1-10 mass ratio, even more preferably 2-7. They are kept stirring at room temperature for 1-4 h, more preferably 2-3 h. The system resulting from this procedure is predominantly nanoparticulated, with dimensions smaller than 200 nm. The rhEGF-rP64k conjugate is bound by electrostatic interactions between the positively charged groups on the surface of the nanoparticle and the carboxylate groups of the conjugate and between the carboxylate groups of the citrate and the amine groups of the conjugate.

In other realization variants tetanus toxoid, KLH or another carrier protein substitutes the recombinant protein rP64k. The present invention also comprises the substitution of the rP64k protein by one or several immunogenic peptides of natural or synthetic origin.

In further realization variants, the rhEGF is substituted by one or several peptides thereof.
- *Adsorption of the rhEGF or peptides thereof and of a carrier protein or peptides thereof to the inorganic particle:*
   A similar procedure to the one described in the previous realization variant is carried out, differing only in that rhEGF and rP64k are added to the dissolution, instead of the rhEGF-rP64k conjugate, in a 1-10 mass ratio, more preferably 2-7. This process requires that a molar ratio of 1-10 of rhEGF per rP64k be maintained, more preferably 2-5 of rhEGF per rP64k. This procedure generates nanoparticles and microparticles that contain on the surface rhEGF and rP64k molecules bound by interactions between the positively charged groups of the particle and the carboxylate groups of the proteins and between the negatively charged groups of the particle and the amine groups of the proteins.

In further realization variants tetanus toxoid, KLH or another carrier protein substitutes the recombinant protein rP64k. The present invention also comprises the substitution of the rP64k protein by one or several immunogenic peptides of natural or synthetic origin.

In further realization variants, the rhEGF is substituted by one or several peptides thereof. The previously described novel particulate systems are formed on a biodegradable inorganic core. This core is bound to autologous antigens, preferably chemical conjugates of recombinant proteins rhEGF and rP64k or individually bound to said proteins or peptides thereof. These systems are sustained by amide type covalent bonds, encapsulation or adsorption on the surface of the nanoparticle. They constitute the base of vaccine formulations for the treatment of cancer, administered are by parenteral route after being dispersed with EDTA and adjusting the pH to 7±0.3.

### Pharmaceutical compositions and treatment methods

The pharmaceutical formulations derived from the present invention are useful for the treatment of epithelial tissue origin cancer. For instance: squamous epithelial cell cancer, small cell lung cancer, non-small cell lung cancer, lung adenocarcinoma, lung squamous carcinoma, hepatocellular, gastric, gastrointestinal, pancreatic, cervical, ovarian, liver, bladder, breast, colon, rectal, colorectal, head and neck and uterine cancer, glioblastoma, salivary gland, kidney, prostate, vulvar, thyroid, anal and penile carcinoma.

They can be used alone or in combination with adjuvants, which potentiates or complements the anti-EGF antibody response generated by them.

Particularly, it is object of the present invention the combination of biodegradable inorganic nanoparticulate systems bound to the rhEGF-rP64k proteins, with oleaginous adjuvants such as incomplete Freund's adjuvants, squalene-based adjuvants, synthetic, mineral, vegetable or animal origin adjuvants. Also, as well as particulate protein adjuvants and liposomes or other adjuvants or systems able to generate or potentiate humoral or cell-mediated immune responses, or the combination of these. The systems described above effectively maintain the previous induction of the anti-EGF antibody immune response, generated with the formulation described in US 8,778,879, patent or with another system that provides similar results.

The combinations described previously favor the chronic treatment of epithelial tissue origin cancer, since treatment with them avoid the accumulation of foreign substances in the body at the injection sites and therefore does not result in administration associated toxicity.

The anti-EGF systems comprised in the present invention use pharmaceutically suitable excipients. These excipients include but are not limited to: water for injection, sodium chloride, phosphorus and potassium salts, calcium chloride, sodium citrate, sodium hydroxide and EDTA. They can be inoculated to patients with epithelial tissue origin cancer in parenteral formulations at protein concentrations from 0.5 to 5 mg/mL and at a dose range between 20-70 µL/kg or 20-70 µg of total proteins per kilogram or up to 5 mg of total proteins, more preferably 30-60 µg/kg. The dose of the inorganic component defined in the present invention to administer, will be between 2-4.5 mg/kg, preferably 3-4 mg/kg, provided that it is within the approved dose range for administration by the intramuscular or subcutaneous route in humans.

The present invention is further elaborated with the following examples and figures. However, these examples should not be construed as limiting the scope of the present invention.

### BRIEF DESCRIPTION OF THE FIGURES

**Figure 1****.** X-Ray Diffraction Pattern: A) Amorphous HAp particles, obtained in the present invention. B) Standard HAp pattern- JCPDS: PDF Ref. 09-0432.
**Figure 2****.** FTIR Spectrum: A) Amorphous HAp particles obtained in the present invention. B) Sodium citrate.
**Figure 3****.** Size of the amorphous HAp particles obtained in the present invention, determined by electronic transmission microscopy. A) Image of the particles recorded at 25000 X magnification. B) Image of the particles recorded at 500000 X magnification. C) Size distribution of the particles.
**Figure 4****.**Thermogram of the amorphous HAp nanoparticles obtained in the present invention.
**Figure 5****.** Size distribution of the HAp-rhEGF-rP64k system covalently bound, determined by the method of dynamic light scattering (DLS).
**Figure 6****.** Characterization of the HAp-rhEGF-rP64k system by means of: A) SDS-PAGE: 1-Molecular mass pattern 2-Positive control of the rhEGF-rP64k conjugate elaborated according to US 8,778,879, invention 3 - HAp-rhEGF-rP64k system. B) Western Blot profile: 1 - Positive control of rhEGF-rP64k conjugate, 2-HAp-rhEGF-rP64k system.
**Figure 7****.** Anti-EGF antibody response of the C57BL/6 mice immunized with the HAp-rhEGF-rP64k system covalently linked and the control group (Montanide-rhEGF-rP64k).
**Figure 8****.** Ratio of antibody subclasses (IgG2b + lgG2c)/lgG1, contained in sera of C57BL/6 mice immunized with the HAp-rhEGF-rP64k systems and the control group (Montanide-rhEGF-rP64k).
**Figure 9****.** Photograph of the effects of the HAp-rhEGF-rP64k and the Montanide-rhEGF-rP64k systems at the injection site in BALB/c mice. A) Effect of the Montanide-rhEGF-rP64k system. B) Effect of the HAp-rhEGF-rP64k system.
**Figure 10****.** Anti-EGF antibody response in C57BL/6 mice immunized with the HAp-rhEGF-rP64k system combined with VSSP and with rhEGF-rP64k encapsulated in liposomes.
**Figure 11****.** Ratio of antibody subclasses (IgG2b + lgG2c)/lgG1, contained in sera of C57BL/6 mice immunized with the HAp-rhEGF-rP64k system combined with VSSP and with rhEGF-rP64k encapsulated in liposomes.
**Figure 12****.** Maintenance of the anti-EGF antibody response of the HAp-rhEGF-rP64k system, with previous induction of the immune response with one or two doses of the Montanide-rhEGF-rP64k system.
**Figure 13****.** Size distribution of the HAp-PI system constructed on the particle, determined by DLS.
**Figure 14****.** Characterization of the HAp-PI system by means of: A) SDS-PAGE: 1- Molecular mass pattern, 2- Positive control of rP64k, 3- Positive control of rhEGF, 4- HAp-PI system and B) Western Blot Profile: 1- Positive control of rP64k, 2- HAp-PI system.
**Figure 15****.** Size distribution of the HAp-CM system, determined by DLS.
**Figure 16****.** Characterization of the HAp-CM system by means of: A) SDS-PAGE: 1- Molecular mass pattern, 2- Positive control of rhEGF-rP64k conjugate elaborated according to US 8,778,879, invention 3- HAp-CM system and B) Western Blot Profile 1- Positive control of rhEGF-rP64k conjugate, 2 - HAp-CM system.
**Figure 17****.** Anti-EGF antibody response in C57BL/6 mice immunized with HAp-rhEGF-rP64k, HAp-PI and HAp-CM systems.
**Figure 18****.** Ratio of antibody subclasses (IgG2b + IgG2c)/IgG1, contained in sera of C57BL/6 mice immunized with the HAp-rhEGF-rP64k, HAp-PI and HAp-CM systems and the Montanide-rhEGF-rP64k control.
**Figure 19****.** Photographic images of the histological sections of muscle tissue extracted at the inoculation site and adjacent muscle tissue of C57BL/6 mice, 10X magnification. * Represents the fibroblastic repair tissue. The mice were treated with: A-C: Montanide-rhEGF-rP64k, D: HAp-rhEGF-rP64k, E: HAp-CM, F: HAp-PI.
**Figure 20****.** Number of lesions observed in the tissues at the sites of injection in C57BL/6 mice immunized with the HAp-rhEGF-rP64k, HAp-PI and HAp-CM systems and the Montanide-rhEGF-rP64k control.

### EXAMPLES

### Example No. 1. Synthesis, characterization and activation of HAp nanoparticles

Following a modified variant of the procedure described by Koroleva M., et al., Russian Journal of inorganic chemistry (2016), 61(6): 674-680), nanoparticles of amorphous HAp coated with sodium citrate were obtained. A solution was prepared under controlled environmental conditions, formed by CaCl₂ 0.05 mol/L and sodium citrate at a sodium citrate /calcium molar ratio of 4:1 (A solution). Next, a sterile B solution was added, formed by NaH₂PO₄ 0.06 mol/L at a flow rate of 1 mL/min, maintaining a Ca/P molar ratio of 1.67. Subsequently, the reaction was adjusted to pH 10 with a dissolution of sodium hydroxide and it was kept stirring for three hours at room temperature. Once the reaction was concluded, it was washed with purified water and separated by filtration using Amicon polyethersulfone membrane of 10 kDa. The obtained solid was vacuum dried at room temperature. Next, they were sterilized with pure steam at 120°C for 30 min and vacuum dried.

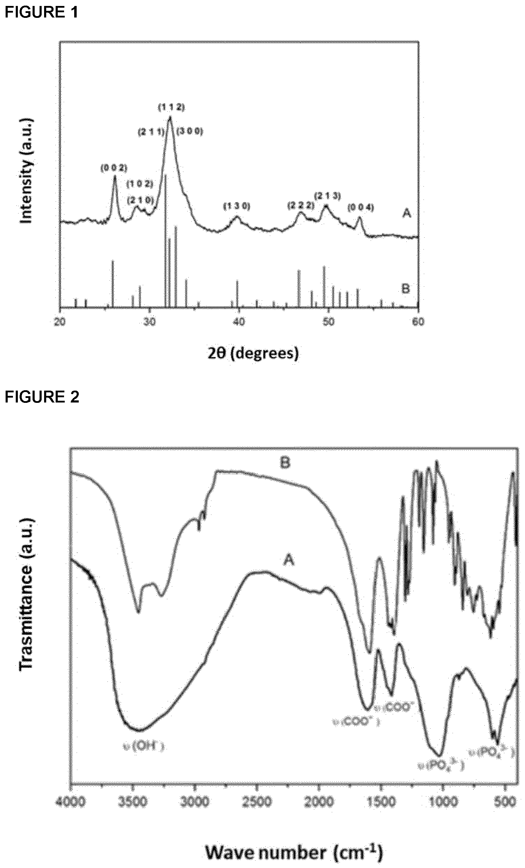

By means of the X-Rays Diffraction assay it was demonstrated that HAp amorphous nanoparticles, with crystallinity degree of 9.3% and crystallite size of 16.5 nm were generated (Figure 1). In this assay copper radiation was used (Cu Kα) in a tube operated at 45 kV and 40 mA. The range was from 10° to 90° with steps of 0.013° at nine seconds intervals.

The Fourier transformed infrared spectra (FTIR) of the nanoparticles (A) and the sodium citrate used as control (B) are shown in Figure 2. The bands observed at 1090, 1030, 962, 604, 561 and 472 cm⁻¹ in the spectrum of the nanoparticles confirmed the presence of the phosphate groups corresponding to the HAp. Also, at 3400 cm⁻¹ a wide band attributed to residual water and the OH⁻ groups was observed. The bands of the carboxylate groups of the citrate were also observed at 1610 and 1413 cm⁻¹ in both spectra, which confirms the presence of this ligand on the surface.

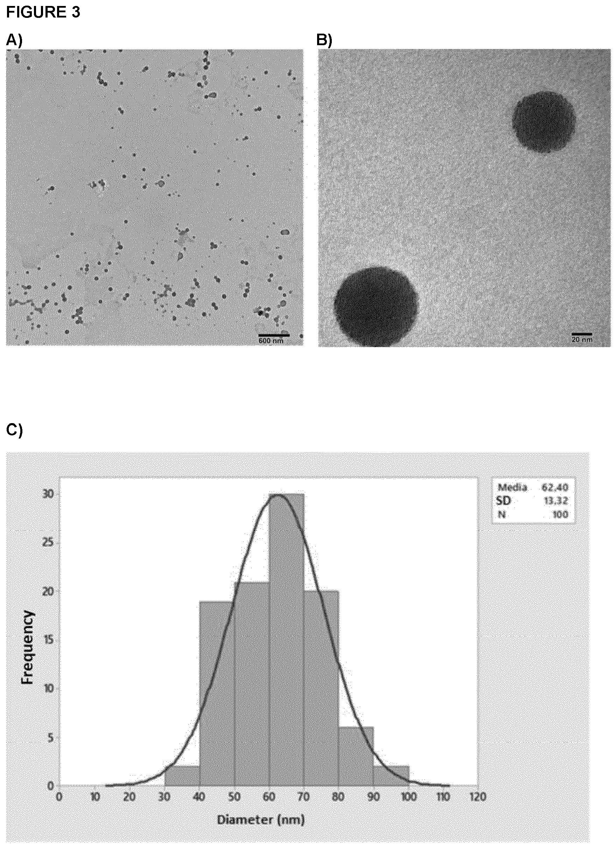

The nanoparticles, determined by Electronic transmission Microscopy, presented a spherical morphology (Figures 3A and 3B), with a size average of 62±13 nm (Figure 3C).

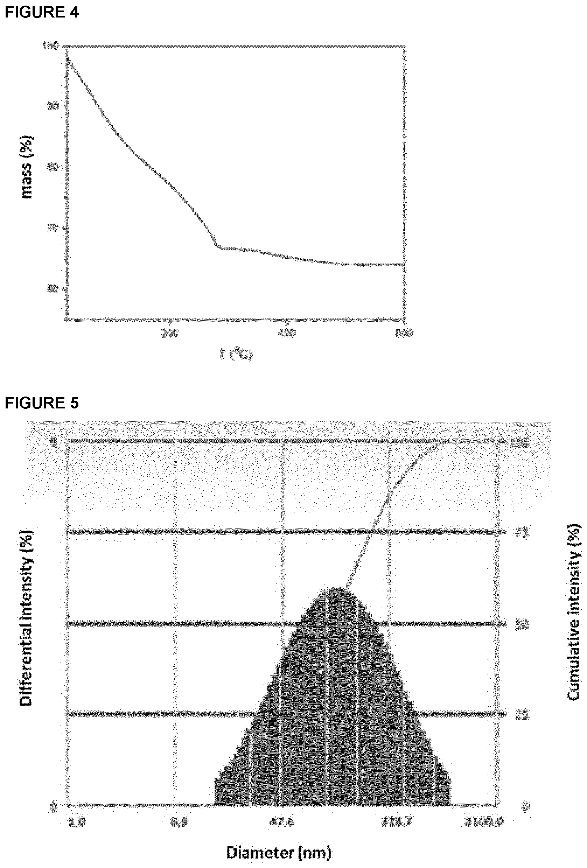

In the thermography a loss of 6.2% of the mass at temperatures higher than 200°C (Figure 4) related to the presence of sodium citrate on the surface of the nanoparticles was found. The range of temperatures analyzed was between 25°C and 1000°C with a heating rate of 20 K/min, using an argon flow of 60 mL/min.

The nanoparticles of amorphous HAp were treated with a sterile dissolution of EDC maintaining a mass ratio EDC/HAp of 2:1 for one hour, under controlled environmental conditions. Then the suspension was centrifuged at 6708 gravities and the excess EDC was removed.

### Example 2. Obtainment of the HAp-rhEGF-rP64k system by means of the covalent bond between the amorphous HAp nanoparticles and the rhEGF-rP64k conjugate

The nanoparticles of amorphous HAp obtained and activated with the procedure described in Example 1 were mixed under controlled environmental conditions, with a sterile solution of PBS, pH 7±0.3, containing the rhEGF-rP64k chemical conjugate obtained with the methodology described in US 8,778,879 invention at a rhEGF-rP64k/HAp mass ratio of 1: 2.5. The suspension formed was kept under shaking at 140 cycles per minute for 2 h at room temperature.

The HAp-rhEGF-rP64k system obtained was dispersed with EDTA, the conditions of the medium were adjusted to a pH of 7±0.3 and protein concentration of 1±0.2 mg/mL, with sterile solutions of PBS and sodium hydroxide.

The DLS assay showed the presence of nanoparticles with mean hydrodynamic diameter of 97.5 nm and a polydispersity index of 0.36. The obtained system was polydisperse with particle diameter ranging from 9 to 366 nm, measured by intensity (Figure 5).

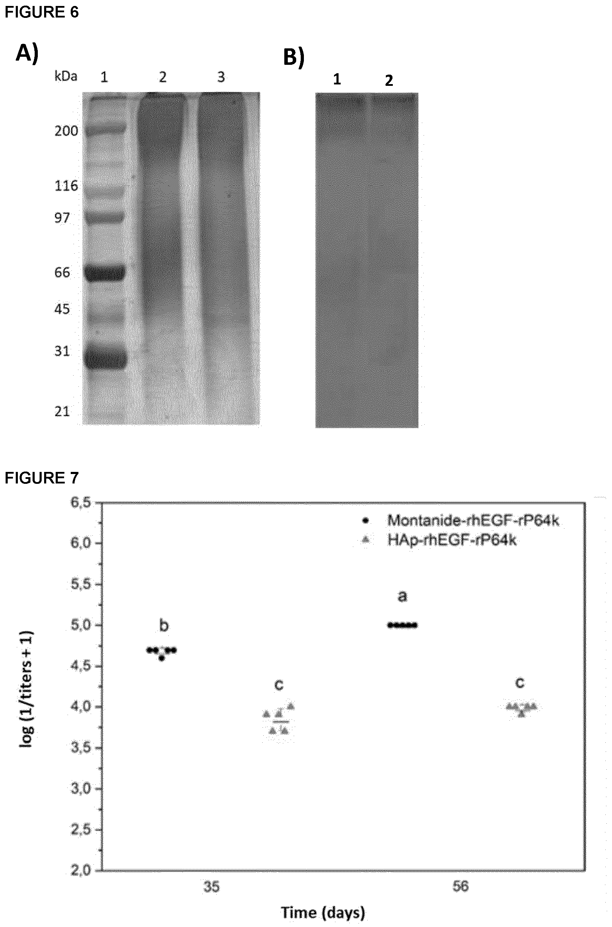

The nanoparticulate system, obtained by means of the previously explained procedure, was characterized by means of SDS-PAGE and Western Blot (Figure 6). The electrophoresis showed the rhEGF-rP64k conjugate had a pattern of bands similar to the one of the positive control (that is, predominant bands with molecular mass equal to or greater than 66 kDa), which indicates the presence of conjugate in the system. In the Western Blot assay, a band similar to the control of the free rhEGF-rP64k conjugate was observed, demonstrating the presence of the rhEGF in conjugates with molecular mass greater than 200 kDa. With this analysis it was proved that the rhEGF-rP64k conjugate bound to the nanoparticles was able to maintain its structural and functional integrity, as confirmed by the fact that it was still recognized by the anti-EGF antibodies.

### Example 3. The HAp-rhEGF-rP64k system generates a humoral response against the EGF without visible adverse effects at the injection sites.

C57BL/6 (n=5) mice were immunized with 63 µg of proteins, contained in the system described in Example 2. A batch of the product obtained with the methodology described in US 8,778,879 invention (Montanide-rhEGF-rP64k) was used as positive control of the assay. An immunization protocol consisting of four doses was applied (days: 0, 14, 28 and 42). Two days before beginning the immunization protocol and on days 35 and 56, the total IgG antibody titers against the EGF were determined in both groups of mice by means of ELISA. The ratio (IgG2b + IgG2c)/IgG1 specific to the EGF was also determined in the immune sera on day 56. The statistical analysis was performed using the test of comparison of means of Kruskal-Wallis, different letters showed statistically significant differences (p<0.05).

The HAp-rhEGF-rP64k system generated anti-EGF antibodies, that were detectable in the immune serum at a dilution of 1/10000 on day 56 (Figure 7). This result demonstrates that the binding of the rhEGF-rP64k conjugate to the particle does not affect the conjugate integrity, and that the HAp potentiates an anti-EGF humoral response in spite of its being significantly lower than the one of the group control. On the other hand, the ratio (IgG2b + IgG2c)/IgG1 showed there were no statistically significant differences in the responses obtained with the HAp-rhEGF-rP64k system and the control (Montanide-rhEGF-rP64k)

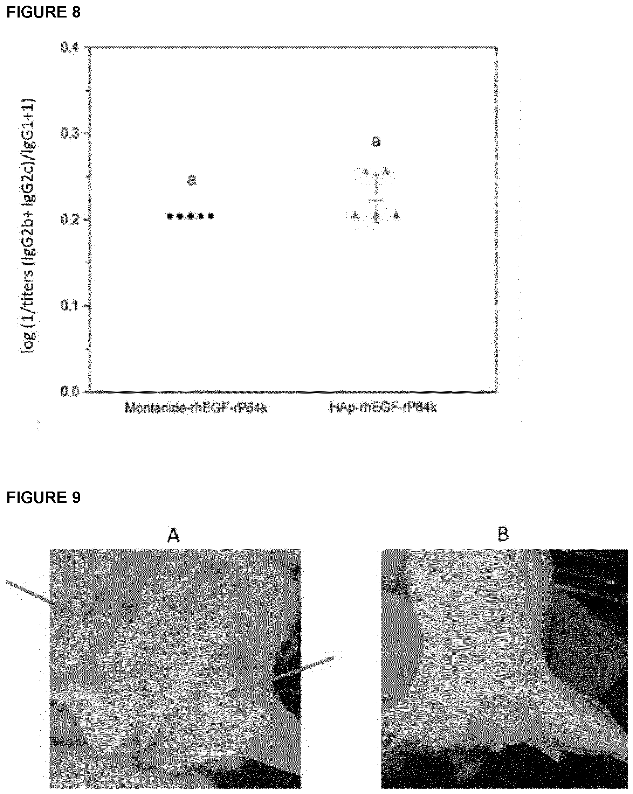

(Figure 8). In both systems, the prevailing responses were of humoral type (Th2), which makes them highly appropriate for the depletion of the EGF, as part of the treatment of epithelial tissue origin cancer.

The effect of the HAp-rhEGF-rP64k system at the injection site was determined. To this, purpose five BALB/c mice were treated with the anti-EGF system described in Example 2 (HaP-rhEGF-rP64k) and five with the Montanide-rhEGF-rP64k control. The vaccination protocol was similar to the one previously described. After concluding the experiment, photographic images of the injection sites of the mice from the two treated groups were taken. As can be seen in Figure 9A, the mice of the control group showed accumulations of mineral oil at the injection sites, caused by Montanide administration. In contrast, in the mice treated with the HAp-rhEGF-rP64k system produced according to the procedure described in Example 2 (Figure 9B), no damage was found at the injection sites. These results indicate that the new system developed in the present invention substantially reduces injuries at the injection site, and thus shows great potential for chronic administration, particularly for the treatment of cancers that require periodic inoculation for several years.

### Example 4. The combination of the HAp-rhEGF-rP64k system with particulate adjuvants generates an anti-EGF IgG antibody response and induces a Th1-type response pattern

C57BL/6 mice divided into three groups of five animals each were used. The animals were immunized in the following way:
Group 1: 63 µg of proteins of the vaccine composition described in US 8,778,879 (Montanide-rhEGF-rhP64k) (Positive control).
Group 2: 63 µg of proteins of the HAp-rhEGF-rP64k system with 100 µg of proteins of the nanoparticulate adjuvant VSSP.
Group 3: 31.5 µg of proteins of the HAp-rhEGF-rP64k system and 31.5 µg of the rhEGF-rP64k conjugate, encapsulated in liposomal vesicles (DRV's) obtained by the methodology of dehydration-rehydration (Kirby and Gregoriadis, Biotechnology, (1984) 2: 979-984).

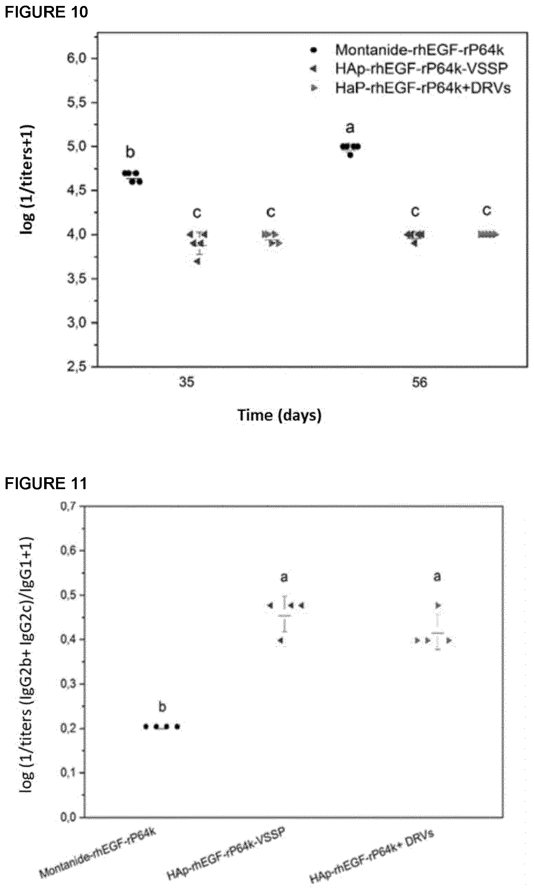

The immunizations were carried out on days 0, 14, 28 and 42. Blood extractions were performed two days before the beginning of the protocol and on days 35 and 56 and the total IgG antibody titers against the EGF in the serum obtained was determined by means of ELISA. The ratio (IgG2b + IgG2c)/IgG1 specific to the EGF in the sera was also determined on day 56. The statistical analysis was performed by means of the test of comparison of means of Kruskal-Wallis, different letters indicate statistically significant differences (p<0.05). The generation of anti-EGF antibodies was evidenced in the three groups studied. The combinations of the HAp-rhEGF-rP64k system with the VSSP and the DRV's liposomes produced antibodies that were detectable in the immune serum up to a dilution of 1/10000 and were same during the two extractions (Figure 10). The existence of statistically significant differences of the combination with respect to the control was demonstrated.

The analysis of the ratio (IgG2b + IgG2c)/IgG1 specific to the EGF on day 56, evidenced both adjuvant combinations exhibited similar responses, and they were both statistically superior as compared to the control (Figure 11). These results demonstrated that the combination of HAp-based formulations with other particulate adjuvants potentiates a specific immune response to the EGF that induces a superior Th1 response pattern, which is the most favorable one for targeted cancer treatments.

### Example 5. The HAp-rhEGF-rP64k system maintains the anti-EGF IgG antibody response previously induced with Montanide-rhEGF-rP64k

Three groups of C57BL/6 mice were used (n=5), which were immunized on days: 0, 14, 28, 42 and 70, with the following immunization schedules:
Group 1. (Control) the mice were immunized with 63 µg of proteins contained in the system described in US 8,778,879 patent (Montanide-rhEGF-rhP64k).
Group 2. The mice were immunized on day 0 with 63 µg of proteins contained in the Montanide-rhEGF-rhP64k system and with same quantity of proteins contained in the HAp-rhEGF-rP64k system on the rest of the immunizations performed.
Group 3. The mice were immunized on days 0 and 14 with 63 µg of proteins contained in the Montanide-rhEGF-rhP64k system and with same quantity of proteins contained in the HAp-rhEGF-rP64k system on the rest of the immunizations performed.

Two days before to the first immunization, the pre-immune serum was extracted and on days 35, 56 and 84 days the extractions of the immune sera were performed and the total IgG antibody titers against the EGF were quantified by means of ELISA.

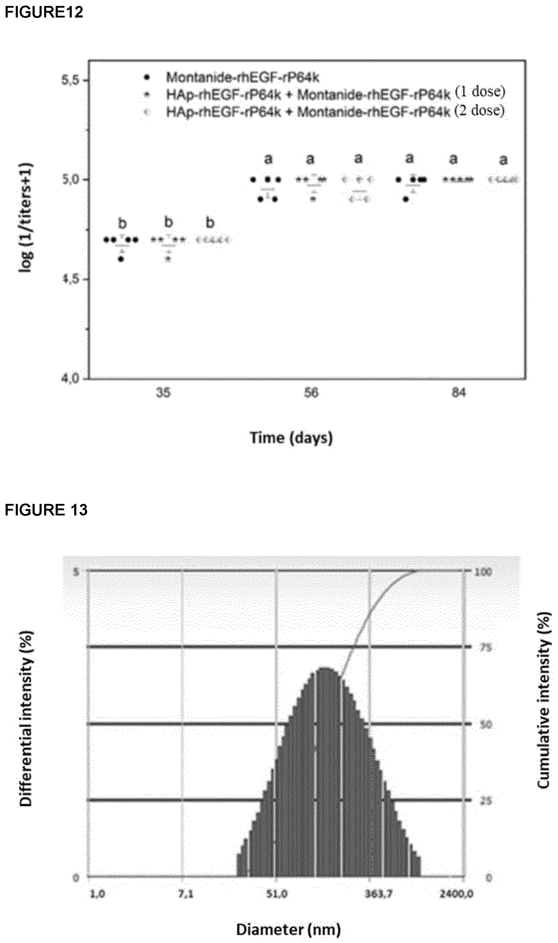

Anti-EGF antibody titers were detectable at the three times studied in the three groups, without statistical significant differences across them, at the 1/50000 dilution on day 35 and at the 1/100000 dilution on days 56 and 84 (Figure 12). The inoculation of the HAp-rhEGF-rP64k system was able to maintain the anti-EGF IgG antibody response induced by the Montanide-rhEGF-rP64k systems, during the time period studied. This result supports the feasibility of substituting Montanide by HAp as adjuvant of the rhEGF-rP64k conjugate in the maintenance phase of the anti-EGF immune response and its suitability in the chronic treatment of epithelial origin cancer.

### Example 6. Construction of the HAp-PI system by means of the covalent binding of the rhEGF and rP64k proteins on the surface of the amorphous HAp nanoparticles

The HAp nanoparticles previously coated with sodium citrate and activated with EDC, obtained according to the methodology described in Example 1, were mixed under controlled environmental conditions with a sterile PBS solution, pH 7±0.3, containing rhEGF and rP64k at a molar ratio of 6 rhEGF per rP64k and proteins/HAp mass ratio of 1: 2.5. The suspension formed was kept under shaking at 140 cycles per minute for 2 h at room temperature. By means of this procedure, a rhEGF-HAp-rP64k system was obtained characterized in that the proteins are bound to the HAp nanoparticle but not to each other. This system was dispersed with EDTA, adjusting the pH to 7±0.3 and the protein concentration to 1±0.2 mg/mL.

Figure 13 shows the particle size profile measured by DLS. The mean diameter was of 111.2 nm, with a polydispersity index of 0.347. The system obtained was similar to the one generated in Example 2 in terms of size, polydispersity and DLS profiles. The above-mentioned aspects indicate when the rhEGF and the rP64k are bound to the HAp nanoparticles, but not to each other, they had a dispersion similar to the one of the rhEGF-rP64k conjugate.

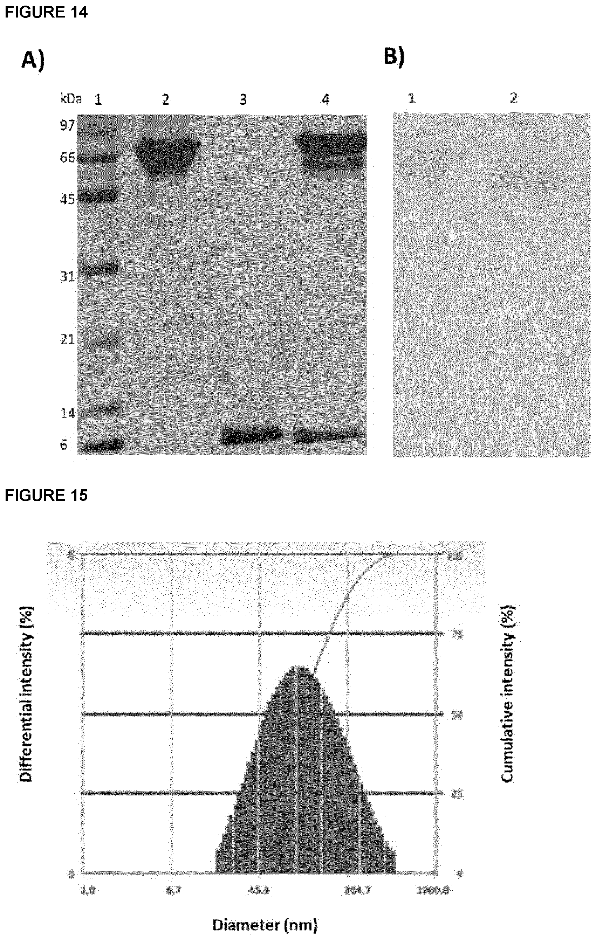

As can be seen in Figure 14A, which shows results from the SDS-PAGE assay, in the lane corresponding to the HAp-PI system, a main band appears at the height of 66 kDa, which is characteristic of the rP64k protein, and another band at similar height than that of the rhEGF control (6 kDa). These results demonstrated that both recombinant proteins were bound to the HAp nanoparticle and that when reduced they maintained their characteristic molecular mass because they were not bound to each other.

As evidenced in Figure 14B there is a band approximately at the height of 66 kDa in lanes 1 and 2, which confirms the presence of the rP64k protein in the HAp-PI system and also that the protein maintains its integrity when it is bound to the HAp nanoparticles.

### Example 7. Construction of the HAp-CM system on the surface of the amorphous HAp nanoparticles by means of the multiple conjugation of the rhEGF and rP64k proteins

The amorphous HAp nanoparticles obtained in Example 1 were treated under controlled environmental conditions for 1 h with a sterile EDC solution, maintaining a mass ratio of 2.7 EDC per HAp. Next, a sterile PBS solution, pH 7±0.3, was added containing the rhEGF and rP64k at a molar ratio of 6 rhEGF per rP64k and proteins/HAp mass ratio of 1:2.5. The suspension formed was kept under shaking at 140 cycles per minute for 2 h at room temperature.

A system formed by HAp nanoparticles coated with both recombinant proteins bound to each other and to the nanoparticles was obtained as a result of this procedure. This system was dispersed with EDTA and the pH was adjusted to 7±0.3. Subsequently, it was diluted with PBS solution until a protein concentration of 1±0.2 mg/mL was reached.

By means of the multiple conjugation previously described, a complex HAp-rhEGF-rP64k system with mean size of 90.2 nm, measured by DLS, and polydispersity index of 0.337 was obtained (Figure 15).

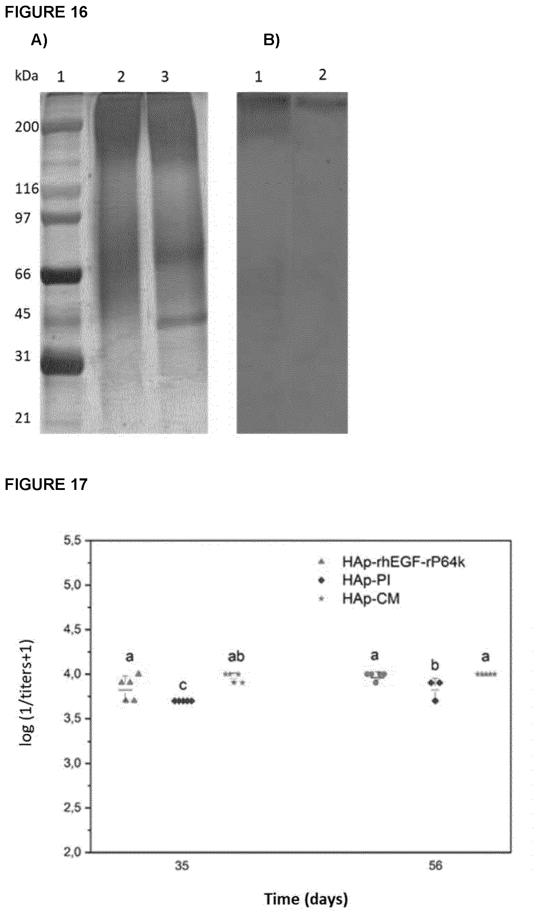

The characterizations performed by means of the SDS-PAGE electrophoresis and Western Blot showed the conjugation of the proteins. A similar product to the rhEGF-rP64k positive control, that recognizes the anti-EGF antibodies, was obtained (Figure 16).

### Example 8. The new systems constructed on the HAp nanoparticles generate anti-EGF IgG antibody responses and produce a smaller number of epidermal lesions at the injection site

Three groups of five C57BL/6 mice each were immunized, with 63 µg of proteins, contained in the following systems:
Group 1: HAp-rhEGF-rhP64k system, obtained according to the methodology described in Example 2.
Group 2: HAp-PI system, obtained according to the methodology described in Example 6.
Group 3: HAp-CM system, obtained according to the methodology described in Example 7.

The immunizations were performed on days 0, 14, 28 and 42. Blood extractions were carried out two days before the beginning of the protocol and on days 35 and 56. The total IgG antibody titers against the EGF in the obtained sera were determined by means of ELISA. The ratio (IgG2b + IgG2c)/IgG1 specific to the EGF in the sera was also determined on day 56. The statistical analysis was performed by means of the test of comparison of means of Kruskal-Wallis, different letters indicate statistically significant differences (p<0.05).

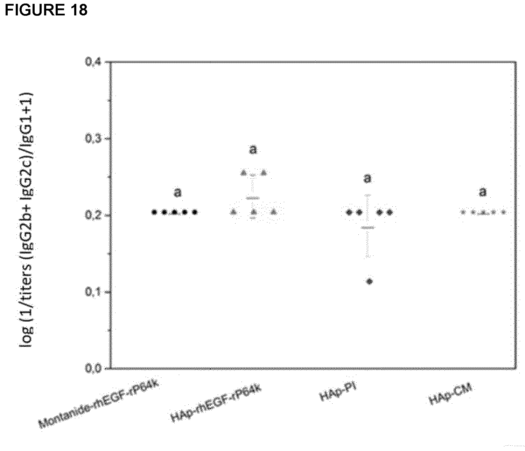

The HAp-rhEGF-rP64k and HAp-CM systems generated anti-EGF antibodies that were detectable in the immune serum up to a dilution of 1/10000 and in the HAp-PI system up to a dilution of 1/8000 on days 35 and 56 (Figure 17). The results obtained by SDS-PAGE and Western Blot were confirmed, (Examples 6 and 7) and it was demonstrated that all the systems constructed on the HAp nanoparticles were immunogenic, although in the HAp-PI system the rhEGF is not in the context of a covalent bond with the rP64k. This system constitutes an evidence that it is feasible to produce systems that generate an anti-EGF response immune without the need of chemically binding the rhEGF to a carrier protein. The analysis of the ratio (IgG2b IgG2c)/IgG1 revealed there were no statistically significant differences in the response produced by them. The prevailing responses were of humoral type (Th2), which makes them appropriate for the depletion of the EGF, as part of the treatment of epithelial tissue origin cancer. (Figure 18).

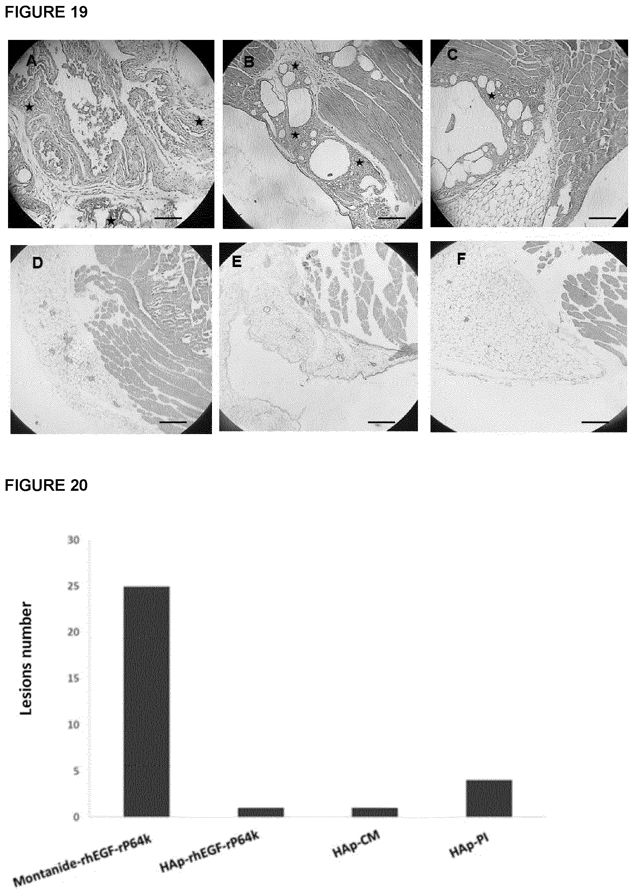

At 120 days after the beginning of the experiment, the C57BL/6 mice were sacrificed and tissue samples from the injection sites were extracted for the anatomopathological analysis, previously staining them with hematoxylin-eosin. The tissue sections were fixed in neutral buffered formalin and they were processed by means of the paraffin embedding method. As can be seen in Figure 19A-C, which shows the results from the control group (Montanide-rhEGF-rP64k), there is a loss of the normal structure of the muscle tissue at the inoculation site. The A zone is characterized by inflammatory infiltrate and dilated blood vessels with discontinuity in the adjacent muscle tissue. In Figures 19B and C, longitudinal (B) and traversal (C) fibers of adjacent muscle tissue at the inoculation site with invasion of a reaction of fibroblastic repair tissue can be observed. However, in Figures 19D-F, which show results from the mice treated with the new systems object of the present invention, a normal structure at the inoculation site and adjacent muscle tissue can be observed. D: HAp-rhEGF-rP64k. E: HAp-CM. F: HAp-Pl.

In each mouse of the three previous groups, the presence or absence of the following lesions was evaluated:
- Discontinuity of the epidermis and lymphocytic infiltration of the dermis.
- Inflammatory infiltrate.
- Invasion of the muscle tissue.
- Vacuolar degeneration of epithelial cells.
- Dilated blood vessels.

A point was added whenever a lesion was found, the values corresponding to each mouse in the groups were added up and the result was graphed.

The study evidenced that the groups treated with the systems developed in the present invention exhibited a very small number of lesions, contrary to that observed in the control group inoculated with the Montanide-rhEGF-rP64k system (Figure 20). This result confirms the visual observations of Example 3.

These results corroborate the low toxicity of the anti-EGF systems based on HAp nanoparticles and the adverse effects in the injection sites caused by the mineral oil contained in the Montanide-rhEGF-rP64k system.

## Claims

1. A vaccine composition to induce an immune response against the epidermal growth factor (EGF), comprising as active principle a system that contains the recombinant human EGF (rhEGF), or peptides thereof, and a carrier protein, bound to a nucleus formed by inorganic nanoparticles.

2. The vaccine composition of claim 1, **characterized by** the inorganic nucleus is formed by salts, oxides or hydroxides selected from the group comprising of: calcium, iron, zinc, magnesium, zirconium, cerium, beryllium, silicon, or the mixture of two or more of them.

3. The vaccine composition of claim 1, **characterized by** the inorganic nucleus is of calcium phosphate.

4. The vaccine composition of claim 1, **characterized by** the calcium phosphate is hydroxyapatite (HAp).

5. The vaccine composition of claim 1, **characterized by** the HAp is of amorphous type.

6. The vaccine composition of claim 1, **characterized by** the HAp has low cristallinity.

7. The vaccine composition of any of claims 1-6, **characterized by** the hydroxyapatite is partially coated by an organic ligand.

8. The vaccine composition of claim 7, **characterized by** the organic ligand is sodium citrate.

9. The vaccine composition of claim 1 **characterized by** the carrier protein is selected from the group comprising:
- cholera toxin B subunit,
- tetanus toxoid,
- KLH and
- P64k of *Neisseria meningitidis.*

10. The vaccine composition of any of claims 1-9 **characterized by** the active principle is on the surface of a HAp nanoparticle.

11. The vaccine composition of claim 8, **characterized by** the active principle is bound to the HAp nanoparticle by means of one of the following methods:
- Covalent bond of the chemical conjugate of the rhEGF or peptides thereof and the carrier protein or peptide with the HAp nanoparticle.
- Covalent bond of the rhEGF or peptides thereof and the carrier protein or peptide with the HAp nanoparticle in an independent way.
- Successive covalent bond of the rhEGF or peptides thereof and the carrier protein or peptide with the HAp nanoparticle.
- Multiple conjugation of the rhEGF or peptides thereof and the carrier protein or peptide on the surface of the HAp.
- Encapsulation or physical binding of the rhEGF and the carrier protein or peptide on the surface of the HAp.

12. The vaccine composition of any of claims 1-9 in combination with other adjuvants that are selected from the group comprising:
- incomplete Freund's adjuvants,
- squalene-based adjuvants,
- synthetic origin adjuvants,
- mineral origin adjuvants,
- vegetable origin adjuvants,
- animal origin adjuvants,
- particulated proteic adjuvants and
- liposomes.

13. The use of the vaccine composition of any of claims 1-10, for the chronic treatment of cancer.

14. A treatment method for a subject in need thereof comprising the administration of a therapeutically effective amount of the vaccine composition of any of claims 1-9.

15. The method of claim 12, wherein a previous immune response induction stage is achieved with another vaccine composition against the EGF.
